Europäisches Patentamt

European Patent Office

Office européen des brevets

(11) Publication number: **0 302 370**

**A1**

# EUROPEAN PATENT APPLICATION

(21) Application number: **88112102.4**

(22) Date of filing: **27.07.88**

(51) Int. Cl.⁴: **A61K 31/71 , A61K 47/00**

(30) Priority: **07.08.87 US 83551**

(43) Date of publication of application:
**08.02.89 Bulletin 89/06**

(84) Designated Contracting States:
**AT BE CH DE ES FR GB GR IT LI LU NL SE**

(71) Applicant: **ABBOTT LABORATORIES**

**Abbott Park Illinois 60064(US)**

(72) Inventor: **Adjei, Akwete L.**
**38770 Red Oak Terrace**
**Wadsworth Illinois 60083(US)**
Inventor: **Vadnere, Madhu K.**
**344 Behm Drive**
**Grayslake Illinois 60030(US)**
Inventor: **Cheskin, Howard S.**
**893 Valley Road**
**Glencoe Illinois 60022(US)**

(74) Representative: **Modiano, Guido et al**
**MODIANO, JOSIF, PISANTY & STAUB**
**Modiano & Associati Baaderstrasse 3**
**D-8000 München 5(DE)**

(54) Erythromycin formulations for oral administration.

㊱ Improved oral formulations for the administration of erythromycin and its derivatives are provided. An oil solution, suspension or emulsion of the erythromycin or derivative, in combination with N-methyl-2-pyrrolidone in a soft gelatin capsule provides enhanced oral bioavailability.

EP 0 302 370 A1

## ERYTHROMYCIN FORMULATIONS FOR ORAL ADMINISTRATION

### Technical Field

This invention relates to liquid, solid or semisolid oral dosage forms for administration of erythromycin antibiotics.

### Background Art

Erythromycin antibiotics are highly effective against a variety of common pathogens and achieve high concentrations in tissues at infection sites, which further enhances their activity against intracellular pathogens, such as legionella. However, difficulty is experienced in achieving adequate bioavailability when administering these drugs orally due to their extremely bitter taste, their limited solubility in water, their susceptibility to acid degradation in the alimentary tract, and like factors well known to pharmaceutical chemists. A variety of approaches have been taken to address each of these problems, including the use of salts and esters of the base compounds, taste masking agents, enteric and other coatings, etc. Nevertheless, there is a continuing need for improved formulations for oral administration of erythromycin antibiotics.

### Disclosure of The Invention

The present invention provides a composition for oral administration of an erythromycin antibiotic, comprising a safe and effective amount of the erythromycin antibiotic, N-methyl-2-pyrrolidone, and a triglyceride oil of mid-chain fatty acids, in a soft gelatin capsule.

By "erythromycin antibiotic" herein is meant erythromycin base and its pharmaceutically acceptable salts and esters, as well as the semisynthetic derivatives of erythromycin known to the art, including without limitation the erythromycin 9-oximes, erythromycin 11,12-cyclic carbonates and $4''$-deoxy-11,12-carbonates, 6-O-methyl-, 11-O-methyl-, and 6,11-di-O-methyl erythromycins, 8-fluoroerythromycin, erythromycin 11,12-cyclic carbamates, erythromycin $4''$-carbamates, and compounds having various combinations of these structural modifications, as well as their pharmaceutically acceptable salts and esters.

By a "safe and effective amount" of the erythromycin antibiotic herein is meant an amount of the erythromycin antibiotic which is within the therapeutic range for the particular compound selected and is effective to treat or prevent infection by susceptible pathogens when administered to a human or animal host in need of such treatment, or a multiple or submultiple thereof. The therapeutic range is the range of dosage between the ED50 and the TD50. Typical safe and effective amounts of erythromycin base range from about 100 mg. in pediatric dosage forms for q.i.d. administration to 500 to 1000 mg. in adult dosage forms for b.i.d. administration. Dosages of erythromycin base of from 250 mg. per capsule to 500 mg. per capsule are especially preferred. Equipotent dosages of other erythromycin compounds are also preferred.

By "triglyceride oil of mid-chain fatty acids" herein is meant a triglyceride composition which is liquid at room temperature (22°C), and which consists primarily of triglycerides of $C_6$ to $C_{18}$ fatty acids, and preferably of triglycerides of $C_6$ to $C_{12}$ aliphatic fatty acids.

By "soft gelatin capsule" herein is meant a gelatin capsule containing a plasticizing agent such as glycerine, sorbitol, 1,2,6-hexanetriol, or equivalent polyols or mixtures thereof, in the capsule wall to render the capsule soft and pliable. Such capsules are particularly well suited for administration of small quantities of liquid compositions in a unitized form.

### Description of the Preferred Embodiments

The preferred erythromycin compound for use herein is selected from the group consisting of erythromycin base, 6-O-methyl erythromycin, and the pharmaceutically acceptable salts and esters thereof.

Most preferred is erythromycin base. The erythromycin compound is preferably dissolved, emulsified or suspended in the triglyceride oil of mid-chain fatty acids by conventional techniques.

One preferred class of triglyceride oils of mid-chain fatty acids is represented by those oils which consist predominantly of glycerol triesters of $C_6$ to $C_{10}$ fatty acids. Such oils can be prepared synthetically by well known techniques, or can be obtained from natural sources by known techniques of thermal or solvent fractionation of suitable natural oils, such as palm oil, to yield a fraction rich in the desired low-melting triglycerides. Such mid-chain fatty acid triglycerides are especially preferred because they have high solubility in and high absorptivity from digestive secretions, so that they improve dispersibility and absorption of the active drug compared with relatively high melting point triglyceride oils having substantial amounts of esters of $C_{14}$ to $C_{22}$ fatty acids. A preferred low melting, low molecular weight triglyceride oil is a low molecular weight fraction of palm oil which is rich in mixed esters of caprylic (octanoic) and capric (decanoic) acids. Such an oil is commercially available as Neobee$^R$ M-5 oil from PVO International, Inc. of Boonton, New Jersey. Other low melting cuts of palm oil are also suitable.

Another preferred class of triglyceride oils consists of triglyceride oils having a high percentage of glycerol triesters of unsaturated or polyunsaturated $C_6$ to $C_{18}$ fatty acids. A preferred example of such an oil is safflower oil, which typically has a fatty acid composition of over 90% oleic and linoleic acids. Triglycerides of these acids are liquid at 20° C, while the corresponding saturated triglyceride tristearin is a waxy solid at room temperature and melts at about 72° C. Other low-melting vegetable oils or low-melting fractions of such oils obtained by conventional thermal or solvent fractionation are also suitable. While such unsaturated or polyunsaturated vegetable oils may offer a cost advantage in formulating compositions according to this invention, they also exhibit a greater tendency to oxidative deterioration, and may require the addition of oil soluble antioxidants, such as tocopherols. In addition, such oils are typically absorbed from the gastrointestinal tract in the form of chylomicrons, which may slow ultimate delivery of the active drug to the blood stream.

In some formulations according to this invention, the triglyceride oil may contain minor amounts of mono- and/or diglycerides to enhance solubility of the components or to enhance emulsification. In other compositions of this invention, depending upon the desires of the formulator, it will be preferable that the oil have a low polarity. In such a case, the preferred triglyceride oils will be low in content of mono- and diglycerides, as well as phospholipids, all of which have significant polarity.

The triglyceride oil is preferably incorporated in the dosage forms of this invention in an amount of from about 75 mg. per capsule to about 750 mg. per capsule, more preferably about 200 mg. per capsule to about 500 mg. per capsule, most preferably about 300 mg. triglyceride oil per capsule containing about 250 mg. erythromycin base.

The N-methyl-2-pyrrolidone is preferably incorporated into the composition in an amount of from about 100 mg. to about 600 mg. per capsule, more preferably from about 150 mg. to about 300 mg. per capsule, most preferably about 200 mg. N-methyl-2-pyrrolidone per capsule containing, for example, about 250 mg. erythromycin base. While not intending to be limited by theory, it is understood that the function of the N-methyl-2-pyrrolidone is to compensate for differences in polarity between the erythromycin antibiotic and the triglyceride oil solvent. In addition to promoting compatibility in the mixture, this compensation permits the erythromycin antibiotic to appear to cell membranes to be more solvent-like in character. Since the triglyceride oil solvent has good membrane permeability, this effect appears to enhance the membrane permeability of the erythromycin antibiotic, and thus improve bioavailability.

The capsule compositions of this invention may also desirably contain minor and pharmaceutically acceptable amounts of emulsifiers, flavoring and coloring agents, antioxidants, buffers and like materials well known to the pharmaceutical chemist. Preferred compositions according to this invention will contain, for example, from about 2 mg. to about 150 mg. of a nontoxic surfactant such as PEG fatty acid esters. Another especially preferred additive in the compositions of this invention is an essential oil. While not intending to be limited by theory, it appears that the inclusion of an essential oil in the formulation alters the biokinetics of the dosage form, favoring more prompt absorption of the active drug. The result is an earlier and higher peak blood level for the drug and sustained subsequent blood levels. Preferred for use in the compositions of this invention are essential oils having from 6 to 12 carbon atoms, preferably from 8 to 12 carbon atoms. Such essential oils include, but are not limited to, eucalyptol, spearmint oil, peppermint oil, cinnamon oil, geraniol, geraniol acetate, and their synthetic equivalents. The essential oil will preferably be incorporated in the compositions of this invention in an amount of about 25 mg. to about 750 mg., more preferably about 100 mg to about 300 mg., most preferably about 150 mg. oil of peppermint per capsule. The capsule compositions of this invention will also preferably contain a pharmaceutically acceptable alcohol solvent for the combination, particularly if oil of peppermint is included in the composition.

By "pharmaceutically acceptable" is meant those compounds which are, within the scope of sound medical judgment, suitable for use in contact with the tissues of humans and lower animals without undue toxicity, irritation, allergic response, and the like, in keeping with a reasonable benefit/risk ratio, and effective for their intended use in the desired composition. Pharmaceutically acceptable alcohol solvents include, without limitation, ethanol and glycerol. The preferred alcohol solvent for the liquid composition is ethanol. From about 10 mg. to about 150 mg., more preferably about 20 mg. ethanol will be employed per capsule.

## Industrial Applicability

The following examples are intended to illustrate the practice and benefits of the invention without being limitative thereof.

## Example I

Soft gelatin capsules of erythromycin base according to this invention are made as follows:

Capsule contents

13.6 grams of PEG fatty acid esters (Emulphor EL-719) are added to 50 grams of a low molecular weight palm oil fraction (NEOBEE M-5) with stirring until the mixture is uniform. To this mixture is added 122.4 grams of N-methyl-2-pyrrolidone, and stirring is continued until the mixture is uniform. Into this mixture is added another 154 grams of the oil and the resulting liquid is mixed well. If it is desired to include an essential oil in the composition, that component is added along with the triglyceride oil. To this mixture is added approximately 180 grams of erythromycin base as the monohydrate, and the combination is mixed slowly until the erythromycin is completely dissolved, and then another 185.59 grams of erythromycin base monohydrate (to a total of 365.59 grams) is added. The solution is again mixed slowly until the added erythromycin is completely dissolved, and then at a moderate speed until the solution is uniform. The resulting composition is assayed for potency and will yield approximately 1000 capsules containing 333 mg. erythromycin base each.

Gelatin capsules

131.5 grams of U.S.P. glycerin, 162.3 grams of sorbitol, 1.54 grams of methylparaben, NF and .39 grams of propylparaben, NF are combined with 280 grams of U.S.P. purified water with good mixing. The resulting mixture is cooled to 10° C with continued mixing, and the cooled mixture is blended with 425.5 grams gelatin, U.S.P. in a chilled blender. The resulting mass is melted at approximately 62° C under vacuum and held at that temperature for encapsulation.

In forming the capsules a sufficient quantity of the erythromycin mixture is encapsulated to ensure that each capsule contains 333 mg. erythromycin by assay. The freshly molded capsules are washed and transferred to trays for drying. Following drying the capsules are inspected and culls are removed. The remaining capsules are washed with acetone, air dried and packaged.

If it is desired to provide the capsules with an enteric coating, a suitable polymer selected for its pH-dependent solubility is applied to the capsules. Selection and application of such polymer coatings is well known to the industrial pharmacist and by itself forms no part of the present invention. For example, in a typical procedure, 30 grams of hydroxypropylmethylcellulose phthalate (HPMCP) Type 55 is dissolved in 190 proof ethanol and U.S.P. purified water (distilled). An appropriate plasticizer for the HPMCP, such as 6 grams castor oil, is added to the solution with thorough mixing. The resulting mixture is applied to the capsules as prepared above in an air suspension coater at a rate of approximately 1 liter of enteric coating solution per kilogram of capsules. The coated capsules are then dried, inspected and packaged.

## Example II

Three compositions were compared in conventional bioavailability studies in dogs. Formulations A and B were prepared in the general manner of Example I. Formulation C was a commercially available erythromycin tablet product providing good bioavailability.

| Formulation | Composition | |
|---|---|---|
| A soft gelatin capsules (enteric coated) | Erythromycin base<br>N-methyl-2-pyrrolidone<br>NEOBEE M-5 oil<br>Ethanol | 250 mg.<br>200 mg.<br>300 mg.<br>20 mg. |
| B soft gelatin capsules (enteric coated) | Erythromycin base<br>N-methyl-2-pyrrolidone<br>NEOBEE M-5 oil<br>Peppermint oil<br>Ethanol | 250 mg.<br>200 mg.<br>180 mg.<br>150 mg.<br>20 mg. |
| C | commercial capsules of enteric coated erythromycin particles containing erythromycin 250 mg. per tablet (PCE , Abbott Laboratories) | |

EP 0 302 370 A1

After overnight fasting, histamine was injected into each dog to stimulate gastric acid secretion, followed in one hour by the appropriate formulation. Blood samples were obtained periodically for 12 hours and assayed for active drug by a standard microbiological method. Variability of the data was within normal limits and no non-absorbers were found in any of the test groups. As shown in the following table, results from these studies demonstrate an approximate two-fold improvement in oral availability of erythromycin base when compared to a leading erythromycin tablet product as control.

| Formulation | $T_{max}$ (hr) | Mean + S.D. | |
| --- | --- | --- | --- |
| | | $C_{max}$ (ug/ml) | AUC (ug/ml x hr) |
| A | 1.67 ± 0.47 | 2.7 ± 1.0 | 9.5 ± 4.5 |
| B | 1.33 ± 0.47 | 3.3 ± 0.6 | 9.0 ± 2.8 |
| C | 1.80 ± 0.40 | 1.9 ± 0.5 | 4.5 ± 1.3 |

**Claims**

1. A capsule composition for oral administration of an erythromycin antibiotic with improved bioavailability, comprising safe and effective amounts of the erythromycin antibiotic, N-methyl-2-pyrrolidone, and a triglyceride oil of mid-chain fatty acids, in a soft gelatin capsule.

2. A composition according to Claim 1 wherein the erythromycin compound is selected from erythromycin base, 6-O-methyl erythromycin, and pharmaceutically acceptable salts and esters thereof.

3. A composition according to Claim 2 wherein the low melting triglyceride oil is a low molecular weight fraction of palm oil comprising mixed esters of caprylic and capric acids.

4. A composition according to Claim 3 which further comprises a pharmaceutically acceptable alcohol solvent.

5. A composition according to Claim 4 wherein the alcohol solvent is selected from ethanol and glycerol.

6. A composition according to Claim 5 which further comprises a pharmaceutically acceptable $C_6$ to $C_{12}$ essential oil.

7. A composition according to Claim 6 in which the essential oil is selected from the group consisting of oil of peppermint, oil of cinnamon, oil of spearmint, eucalyptol, geraniol, geranyl acetate, and synthetic equivalents thereof.

8. A composition according to Claim 1 which contains from about 100 mg. to about 1000 mg. of the erythromycin compound.

9. A composition according to Claim 1 which contains from about 100 mg to about 600 mg N-methyl-2-pyrrolidone.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 89, 1978, page 362, no. 152660r, Columbus, Ohio, US; V. SPRINGOLO: "Bioavailability of formulations containing erythromycin base or erythromycin stearate in gastric-resistant soft gelatin capsules", & BOLL. CHIM. FARM. 1978, 117(2), 113-21 | | A 61 K 31/71<br>A 61 K 47/00 |
| A | FR-A-2 206 943 (SUMITOMO CHEMICAL CO., LTD)<br>* Page 4, lines 28-33; claims 1-12 * | | |
| A | US-A-4 612 187 (TAKEO LIJIMA et al.)<br>* Claim 1 * | | |
| A | EP-A-0 032 825 (MALLINCKRODT INC.)<br>* Claims 1-10 * | | |
| A | EP-A-0 107 085 (WARNER-LAMBERT CO.)<br>* Claims 1-5 * | | **TECHNICAL FIELDS SEARCHED (Int. Cl.4)** |
| A | US-A-4 079 128 (S.-L. LIN et al.)<br>* Column 3, lines 10-22; claim 1 * | | A 61 K |

The present search report has been drawn up for all claims

| Place of search | Date of completion of the search | Examiner |
|---|---|---|
| THE HAGUE | 22-11-1988 | TZSCHOPPE,D.A. |